# EUROPEAN PATENT APPLICATION

(11) **EP 4 001 346 A1**
(43) Date of publication of application: **25.05.2022**
(21) Application number: 20208958.7
(22) Date of filing: 20.11.2020
(51) Int. Cl.: C08J 5/18, C08J 7/04, B29C 59/00, C09D 183/04

(54) **FABRICATION METHOD OF AN ELASTOMER WITH TOPOGRAPHICAL STRUCTURE FORMED BY THE BREATH FIGURE TECHNIQUE**

(71) Applicant: ETH Zürich, 8092 Zürich (CH)
(72) Inventor: FERRARI, Aldo, 8057 Zurich (CH); MAZZA, Edoardo, 8112 Otelfingen (CH); HOPF, Raoul, 3011 Bern (CH); KOUROUKLIS, Andreas, 8050 Zurich (CH); WU, Xi, 8049 Zurich (CH)

(57) **Abstract**

A fabrication method of an elastomer breath structure comprises the steps of applying a liquid elastomer (21) onto a substrate, an initial curing of the liquid elastomer (21) on the substrate partially into a partially cured polymer (22), condensation of water droplets (40) from the environment (41') onto the partially cured polymer (22), and finishing curing of the partially cured polymer (22) with parallel removal of the water droplets (40) creating surface dimples (33) on the cured elastomer (23).

## Description

### TECHNICAL FIELD

The present invention relates to a fabrication method of elastomer breath structure and an chemically or biologically treated and modified elastomer breath topographical structure, a patterned surface produced with it and a driveline as application product.

### PRIOR ART

WO 2014/036290 A1 discloses a drug delivery device and a method of making such a drug delivery device based on single and multi-layer polymer films made by a breath figure technique having therapeutic agents associated therewith. For example, the devices may be a dual layer polymer film wherein the first layer includes a therapeutic agent incorporated into it by spin coating the first agent with a polymer solution and CH₂Cl₂ as solvent and the second agent is incorporated into the second layer by loading the agent into pores of the second layer after it is spin coated onto the first layer. In some cases one layer provides a burst release and the second layer provides a slow release drug delivery profile. The devices may take on the form of a surgical mesh with a slow release therapeutic drug.

EP 1 887 388 A2 discloses a type of optical film, processes for making such a film, and a backlight device containing the film. The film includes a substantially monolayer arrangement of beads partially submerged in a binder. Such a film is achieved by applying to a transparent substrate a coating comprising a carrier, an aqueous dispersible or soluble polymeric binder and a bead population of desired refractive index and low polydispersity, wherein the binder is selected to have a refractive index that substantially matches that of the beads upon removal of the carrier, and wherein the relative proportions of the coating components is sufficient to provide a substantially mono layer arrangement of the beads that is partially submerged in the binder upon removal of the carrier; and then removing the carrier from the coating to provide a substrate coated with a substantially mono layer arrangement of the beads that is partially submerged in the binder. Useful solvents for the polymer suspension process are those that dissolve the polymer, which are immiscible with water and which are readily removed from the polymer droplets such as, for example, chloromethane, dichloromethane, ethylacetate, vinyl chloride, methyl ethyl ketone, trichloromethane, carbon tetrachloride, ethylene chloride, trichloroethane, toluene, xylene, cyclohexanone, 2-nitropropane and the like.

A further application of the breath figure technique can be found in US 2017/096545 A1, where a solid state dye laser is prepared with the laser dye being incorporated in pores of the copolymer membrane.

The technology is broadly used as there exists a wikipedia article relating to Breath-figure self-assembly; https://en.wikipedia.org/wiki/Breath-figure self-assembly mainly edited and updated in 2018 and 2019. By casting a thin layer of solvent on the already cured polymer surface, the material will be softened. In a humid environment of limited control, water droplet condensation will be induced spontaneously due to the heat removed by the evaporation of solvent. The condensed droplets indent the soft surface by the effect of gravity and surface tension. The resultant topography is a honeycomb structure with sub-micrometer, mono-sized imprints. Endothelial cells (ECs) can sense and respond to the surface roughness associated with these topographies.

This breath figure technology is casting a solvent on the polymer and evaporates it to fabricate the uniform honeycomb topography with unit size < 10um. Since the process in humid environment is under little control unit sizes > 10um are seldomly produced.

Different solutions of driveline development become the starting point of percutaneous driveline infections, in short DLIs. The number of patients experiencing such infections are steadily increasing. Such DLIs are detected via either superficial symptoms such as redness, increased temperature, and purulent discharge at the exit site or as a clinically apparent abscess at the lower layers of the soft tissues (e.g. fascia and muscles).

### SUMMARY OF THE INVENTION

Based on this prior art it is an object of the invention to provide a better controlled and better performing fabrication method of a breath topological structure, providing the possibility of creating unit sizes > 50um, allowing for secondary shallow craters, and/or imprints with certain size distributions which are features unkown with the prior art methods. There, the indented wells are densely packed without control of the inter-well distance.

The object of the invention is achieved with an improved fabrication method according to the features of claim 1.

A fabrication method of an elastomer breath structure or elastomer breath topological structure comprises the steps of applying a liquid elastomer onto a substrate, initial curing the liquid elastomer on the substrate partially into a partially cured polymer, condensation of water droplets from the environment onto the partially cured polymer and finishing curing of the partially cured polymer with parallel or sequential removal of the water droplets creating surface dimples on the cured elastomer.

Within the fabrication method the condensation step can comprise controlling the original environment of the partially cured polymer as a humid chamber atmosphere with a relative humidity of between 20 and 100%, especially 40% and 100% and further especially 60 to 90%.

The humid chamber atmosphere can be controlled that the surface covered by water droplets is between 10% and 80%, especially 20 to 50%, of the polymer covered substrate surface.

The initial curing step of the liquid elastomer on the substrate into a partially cured polymer can be configured to obtain a partially cured polymer with a surface tension such that condensed water droplets sink in into the previous undisturbed surface by a depth of 1 to 100% before the finishing curing step evaporates the sunk in water droplets. In an application, the water droplets may have a size between 10 and 100 micrometres and sink in into the previous undisturbed surface by a depth of 1 to 10 micrometres before the finishing curing step evaporates these sunk in water droplets.

The aspect ratio of well width to well depth can be between 30 to 1 and 10 to 1.

The humidity and temperature of the humid environment (41') can be controlled within the condensation step and the finishing curing step. By increasing humidity, the droplet size is increased, while inter-droplet distance is decreased. These results in larger well diameter and smaller inter-well distance. Raising the temperature increases the curing speed, resulting in smaller diameter.

The final curing step which comprises the removal of droplets can be executed in different manners, especially through evaporation, washing or centrifuging to reveal the topography of the breath structures onto the elastomer.

The application step can be preceded by a step of mixing one or more liquid polymers, since a mix of different polymers provide more flexibility in the choice of the resulting well structure.

Such a mixing step can comprise mixing of one or more liquid polymers, especially room temperature vulcanizing silicones or silicones curing at temperatures between -10 and +60 degrees Celsius, optionally with polymers and copolymers having a different degree of miscibility.

The application step of the liquid polymer on the substrate can be conducted by a process from the group encompassing spin-coating, dip-coating, air spray or 3D-printing.

The finishing curing step can be followed by a chemical or biological treatment step to apply predetermined chemical compounds or biological cells into the surface dimples and on the in-between dimple surface which are steps towards a direct application of the created breath surface.

A patterned surface of a polymer can be fabricated according the above cited method having an aspect ratio of well width to well depth between 30 to 1 and 10 to 1.

Based on the prior art it is an aim of the present invention to provide percutaneous drivelines facilitating skin integration and reduce DLIs.

The object of the invention is tackled with an improved topographical modification of the percutaneous driveline according to the features of claim 13.

Such a percutaneous driveline is coated at least adjacent to the percutaneous portion with a patterned surface according to the invention, wherein predetermined chemical compounds or biological cells are in the surface dimples and/or on the in-between dimple surface.
Similarly, an effective transfer of topographical cues that secures the infection-free application of percutaneous drivelines will also benefit a wide range of medical devices that require access to blood (i.e. dialysis), different tissues (i.e. tissue monitoring), body cavities (i.e. prosthetic urethra), and internal prosthetic parts (i.e. corneal implant).

Focusing on a different set of applications, the transfer of tunable topographical cues on biomedical devices can alleviate the negative effects from adverse foreign body (FB) responses. The present fabrication method uniquely fulfills scale up requirements when covering large surfaces of biomedical devices (e.g. the entire luminal surface of cardiovascular devices). Similarly, the invention manages to successfully transfer the selected topographies on non-conventional three-dimensional (3D) surfaces which are regularly encountered in biomedical equipment and instrumentation.

To this end, the claimed method of imprinting selected topographies offers a solution to obtain endothelialization over artificial surfaces with complex 3D geometries. Overall, this free-form imprinting technology constitutes a new method to efficiently introduce breath structures on surfaces that are engineered to reduce tissue morbidity and improve the life style of the patients.

The surfaces of different biomedical devices (e.g. drivelines and luminal surfaces) can be modified with the method according to the invention with selected surface chemistry and rationally developed microscale surface topography. Specifically, a silicone formulation that favors low infection, enhanced tissue integration, and non-cytotoxic behaviour is used. The incorporated silicone layer covers substrates of various geometries, that span from thin copper wires to flat membranes to curved tubes and is subject to further modification by imprinting selected surface topographies. The imprint procedure develops by a free-form process following the condensation of water droplets on top of the uncured silicon surface. This method obtains selected topographical patterns of breath morphology that resembles micrometer-sized circular wells. In this manner, fine control of the diameter, depth and distance between the wells of the breath topography by just tuning the curing time preceding the condensation step is obtained.

The effects of breath topography can be alternatively modulated with the incorporation of selected chemical and biological molecules (e.g. polymers, DNA molecules, peptides, antibodies, matrix proteins). Orthogonal control of breath topography and presented chemistry can expand the combinations of instructive cues to account for the different mechanisms involved in a variety of tissue responses involved such as wound healing, skin integration, and endothelialization.

The present invention uses a known silicon formulation as the substrate material. However, then a selected topography is applied on top of the silicon in contrast with prior art lacking control of surface topography, especially on the inter-well distance.

According to the present invention a random spatial distribution of features with disperse imprint sizes and depths in the micrometer range is achieved. The unit imprint can house 1-2 endothelial cells. The invention also differs from the existing condensation-imprint technology by the absence of any organic solvent. The condensation is enforced during the silicone curing process, in which the silicone undergoes a liquid-to-solid transition process. The curing process is hence divided into two sequential parts: initial curing and finish curing. The condensation is induced by controlling environmental temperature and humidity instead of solvent evaporation.

The present invention overcomes problems inherent to current methods for imprinting breath topography on synthetic biomedical surfaces, offering a new therapeutic avenue by triggering select tissue responses in the human body. Instead of using regular chemical and biological factors, the present technology harnesses the physical characteristics of the imprinting breath topography to promote therapeutic cell and tissue responses. That is, the breath topographies produced according to the invention can exert physically instructive signals to biological tissues with therapeutic benefits for the patient.

The present invention presents a surface topography with certain size distribution and (diameter-to-thickness) aspect ratio, which can be controlled by the two-step curing method. The size distribution and tunable aspect ratio suits better in cell cultivation, because size distributions and large aspect ratio resembles more closely the natural morphology of most cells. The present invention also includes a new degree of freedom, namely the inter-well distance, which is not presented in the prior arts. The tuning of inter-well distance will control the percentage coverage of the topography on the surface, which can be further treated for culturing cells individually or in clusters.

Further embodiments of the invention are laid down in the dependent claims.

The process according to the invention is different to the prior art in both chemistry and physics. From the chemistry point of view, there is no reagent (e.g. polymer) dissolution employed in the method according to the invention. In contrast, the method uses a two-step curing process (before and after droplet formation) to control the interfacial force balance between the water droplet and the curing polymer without dissolving neither the curing polymer nor the curing polymer substrate. In effect of these steps in the fabrication process, the present method is more economic, environmentally friendly, toxicity-free, and sustainable. From the physics point of view, what prior art utilize is a liquid-liquid interaction. In contrast, the present method relies on a liquid-(soft)solid interaction.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows a flow chart of an embodiment of the method according to the invention.
- Fig. 2A, 2B and 2C: shows three schematical cross-section side views of the polymer handling after different steps of the method according to Fig. 1;
- Fig. 3A, 3B and 3C: shows three schematical cross-section side views of the polymer handling after different steps of the method according to Fig. 1;
- Fig. 4: shows a schematic side view diagram with topography parameters;
- Fig. 5: shows schematically different configurations of the device setup after the final treatment step; and
- Fig. 6: shows a microscopic image of a patterned surface after application of the invention.

### DESCRIPTION OF PREFERRED EMBODIMENTS

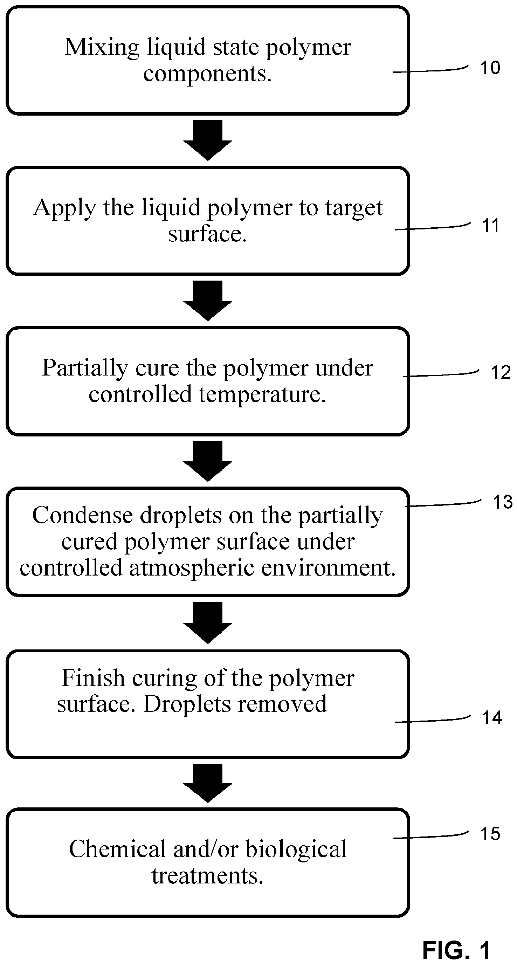

Fig. 1 shows a flow chart of an embodiment of the method according to the invention and discloses selected materials and modes for the breath figure formation according to an embodiment of the invention.

The method starts with a mixing step 10. The used components of room temperature vulcanizing (RTV) silicones are sufficiently mixed. In other embodiments silicones are mixed which crosslink to form elastomers at lower or higher temperatures, especially between -20 to +60 degree C. Different temperature vulcanizing silicones are associated with working solutions of variant viscosity, mediating the efficiency of coverage of the coated surface and the final thickness of the spread silicone layer. Higher temperature will in general result in a faster curing, hence reducing the fabrication time, while lower temperature in general slows down the curing process, results in a longer handling time, more homogeneous spread on surfaces.

The RTV cures by polyaddition reaction of silicone reactivegroups (-SiH) with silicone unsaturated polymers (such as -Si-CH-CH2), under the platinum catalyst environment. Component A (containing platinum catalyst) and component B are mixed in various ratios (including 1:1 ratio). The mixing ratio and the curing temperature affect the extend of crosslinking during the first step of curing. In turn, this influences the interfacial force balance and the associated droplet-induced deformation, which together determine the size and aspect ratio of the breath structures obtained.

Similar to RTV silicones, other silicone-based polymers can be recruited to promote the formation of breath figures by using the described method. Polydimethylsiloxane (PDMS), PDMS-based (e.g. Polydimethylsiloxane-diacrylamide, Poly(dimethylsiloxane), diglycidyl ether terminated) and PDMS copolymers (e.g. Poly(dimethylsiloxane-coalkylmethylsiloxane), Poly[dimethylsiloxane-co-(2-(3,4-epoxycyclohexyl)ethyl)methylsiloxane], Poly(dimethylsiloxane-co-methylphenylsiloxane)) can be used separately or mixed in different combinations with RTV silicones to affect the characteristics of the breath figures.

RTV silicones can also be used in the presence of other polymers such as blends of diblock-copolymers (e.g. polysiloxane-poly(oxyalkylene), poly(dimethylsiloxane)-poly(oxydimethylsilylene), polycarbonate-PDMS) multiblock copolymers, homopolymers, which demonstrate different degree of miscibility with RTV silicones. Similarly, RTV silicones can be controllably blended with different type of surfactants that bear a miscible hydrophobic part and a polar counterpart. The number density, the chemistry, and the architecture of these secondary components correlates with the rate of crosslinking, the mechanical properties, and the surface tension of the silicone elastomer, and eventually directing the physical and chemical characteristics of the breath figures. The volume fraction (0.01-1% v/v), the chemistry (molecular similarity with RTV) of these secondary components will affect the degree of partial curing at the two-step curing process before and after water condensation. The distribution of surfactants in the partially cured elastomer will also affect the Y_{sw} and consequently the shape and the size distribution of the breath structures.

Other suitable formulations involve blends of RTV silicones with different components of organic/polymeric (e.g. polystyrene, poly-ethylene glycol, Teflon, poly(lactic-co-glycolic acid) and inorganic (e.g. silicon, metallic, carbon-based) micro- and nano-particles (or powders). The number density, the chemistry, and the architecture of the used particles can be selected to control the rate of crosslinking, the mechanical properties, and the surface tension of the silicone elastomer and consequently tune the characteristics of the breath figures. The volume fraction (0.01-1% v/v), the chemistry (hydrophilic) of these particle components in the partially cured elastomer will affect the γ_{sw} and consequently the shape and size distribution of the breath structures.

The above mentioned pre-mixed liquid state polymer component(s) are applied in application step 12 to a substrate. RTV silicones (and other silicone based polymers) used in the method for breath figure formation can be cast on different materials (e.g. wax, gypsum, low melt alloys, metals, urethane, polyester resins, polystyrene and other substrates) composing flat (2-D) and curved (3-D) surfaces.

The application step 11 itself can be executed through various methods of deposition of RTV silicones, e.g. spin-coating the select material (e.g. RTV silicone) over different surfaces with various centrifugation speeds (100-2000 rpm) and centrifugation time. The speed and time is tuned to control the thickness of the deposited layer with further effects on the deformability of the silicone elastomer and finally the breath figures topography. The thickness of the coating layer 22 varies between tens of micrometers to a few hundreds of micrometers. As an example, a 60um thickness coating was achieved by 700rpm for 90s on a flat surface. In general, an increased centrifugation speed and increased time will result in a smaller coating thickness. The reduced thickness in turn increases the boundary effect of the underlying substrate 51, oftentimes stiff material, hence decrease the deformability of the silicone elastomer.

In other embodiments the application step 11 is provided with casting and dip-coating of RTV silicones on-top of such select surfaces. The drawing speed of dip coating or the other relevant parameters of casting can be tuned to control the thickness of the deposited layer.

In general, a faster drawing speed results in a thinner coating. The thickness of the layer varies between tens of micrometers to a few hundreds of micrometers.

It is also contemplated that the application step comprises a 3-D printing of RTV-silicones. Here, in case of two or more polymers, it is possible to print and mix the polymers in situ instead of the mixing step 10.

The application step 11 can be summarized in that the aforementioned liquid polymer mixture is applied to a target surface, by the means of spin coating, dip coating, air spray or other liquid deposition methods. The thickness of polymer layer is controlled accordingly by the deposition process parameter, e.g. rotational speed in spin coating, drawing speed in dip coating, air flow rate in air spray, etc.. The thickness of the layer varies between tens of micrometers to a few hundreds of micrometers.

The curing itself happens in two curing steps. The application step 11 is followed by an initial partial curing step 12, within which the aforementioned polymer surface layer is partially cured under controlled temperature. The curing target can be set by fixing amount of time, or in-situ testing of mechanical property on a reference piece. The mechanical property criteria, including but not limited to Young's modulus and viscosity, affect directly the diameter and the depth of the breath topography. The initial Young's modulus (very small) and viscosity (6000 mPa*s) in general result in smaller size (<10um), small inter-well distance 72 (more closely packed) and lower diameter 71 to depth 73 aspect ratio (<10:1). On the other extreme, the almost cured Young's modulus (500kPa) and viscosity (very high) will result in large size (>100um), large inter-well distance 72 and large diameter 71 -to - depth 73 aspect ratio (>30:1). In-between conditions can be seen as interpolation between two conditions. The curing target can be continuously selected to meet specific environment.

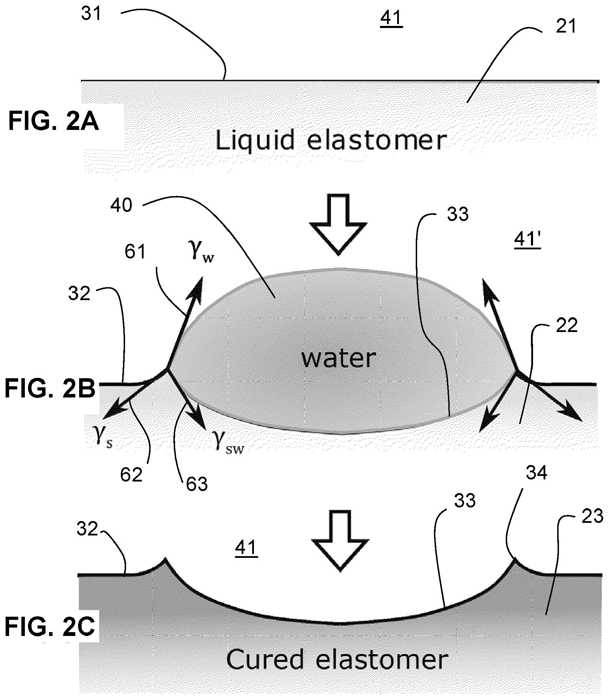

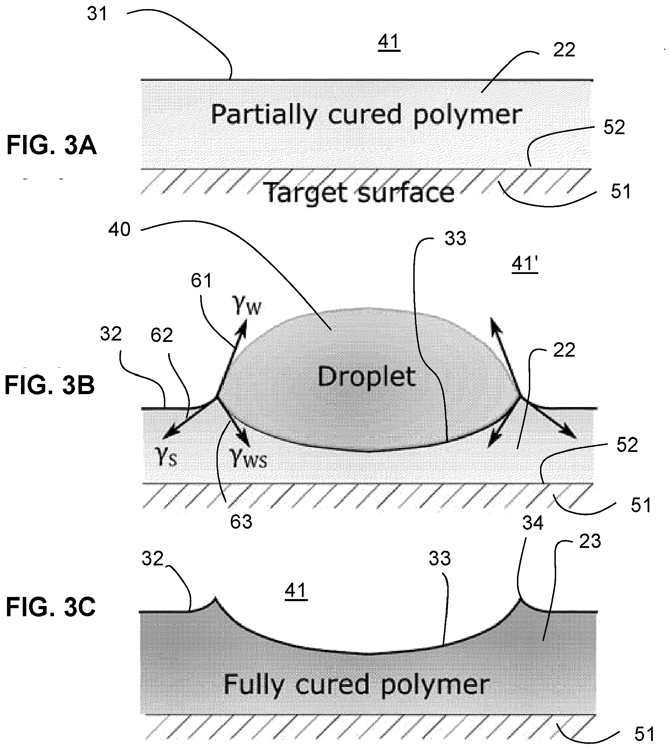

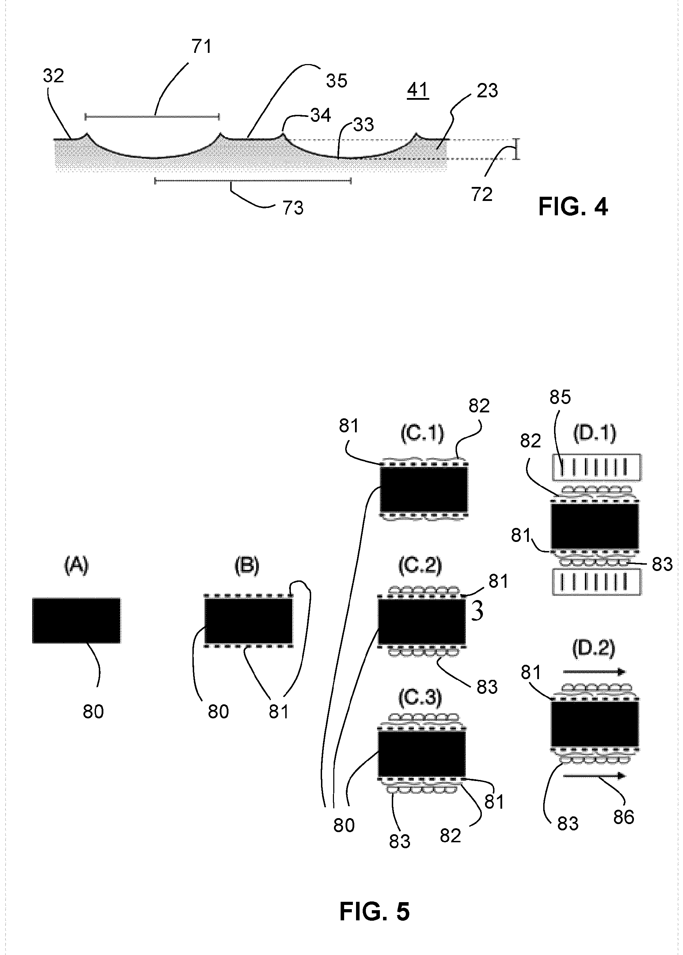

Fig. 2A to Fig. 2C show schematic diagrams of parts of the patterning mechanism using the method steps mentioned in connection with the description of Fig. 1. Fig. 2A simply shows a liquid polymer layer 21 of predetermined thickness, being larger than the intended indentation or well depth 72 (see Fig. 4) being separated by the flat liquid polymer surface 31 vis-à-vis the environment 41, ambient air. The surface 31 can also be curved, especially, if the underlying substrate 51 is not flat like in Fig. 3A but curved to obtain a curved polymer element. Insofar Fig. 2A shows a situation after the application step 11 and before the initial partial curing step 12.

After the initial partial curing step 12 (not shown in Fig. 2A, 2B or 2C) droplets of various sizes 40 of previously vaporized water are formed by condensation onto the semi-cured elastomer within a chamber of controlled and high relative humidity, step 13. Here the polymer material is already mentioned as partially cured polymer 22. The originally flat surface 31 of the polymer surface is deformed by the water droplet 40 in deposition into an inside well surface 33 and an outside well surface 32. The two surfaces are separated by a circumferential edge 34 (shown in Fig. 2C) running at the three media contact points, i.e. between the environment air 41, the water droplet 40 and the partially cured polymer 32. The relevant vector 61, 62 and 63 are described in connection with Fig. 3A to 3C. The chamber humidity also affects the droplet diameter 71, and inter-well distance 72. Provided the same degree of initial curing step12, a higher humidity In step 13 will result in larger droplet diameter 71 and smaller inter-well distance 72. Chamber temperatures affect the speed of curing at the second stage of finish curing. A higher temperature accelerates curing, hence result in smaller well diameter 71.

Fig. 2C finally shows the situation after the polymer 23 is fully cured. The removal of droplets 40, i.e. through evaporation, reveals the topography, i.e. surface portions 32 and 33 with intermediate well edges 34.

Fig. 3A to Fig. 3C show schematic diagrams of parts of the patterning mechanism using the method steps mentioned in connection with the description of Fig. 1. Fig. 3A shows a cross-section view of liquid polymer already been applied on the target surface 52 of target 51. and partially cured, i.e. a partial cured polymer 22.

Fig. 3B shows water droplets 40 which are already condensed on the polymer surface 31, therefore the polymer surface is now a polymer surface 32 outside the condensation and a polymer surface 33 inside a well. By the means of surface tension, the droplets 40 deform the partially cured polymer 22. The components of surface tension are indicated as Yw (surface energy between liquid and air) 61, γ_{S} (surface energy between solid and air) 62 and γ_{WS} (surface energy between liquid and solid) 63. The term "solid " is used for the polymer, since it is already the partially cured polymer 22. Finally, Fig. 3C shows the situation after the polymer is fully cured. The removal of droplets 40, i.e. through evaporation, washing or centrifuging reveals the topography of the breath structures onto the elastomer, i.e. surface portions 32 and 33 with intermediate well edges 34.

Fig. 4 shows a schematic side view diagram with topography parameters; possible when applying the method according to an embodiment of the invention like in Fig. 1 with specific breath figures parameters. The parameters used to characterize the resulting breath topography are: diameter 71, depth 73 and inter-well distance 72 of the breath figures. The predetermined well diameter 71 of the breath figures recorded should be in the mean range between 5µm to 80 µm. The formation of breath structures of diameter 71 >10um overcomes a feature (product) limitation that previous methods have not been able to achieve. The well depth 72 of the breath figures recorded should be in the mean range between 0.5 µm to 15 µm, which in turn results in an diameter-to-depth aspect ratio different than the prior art which is related to the humidity and the chemical nature of water. The inter-well distance between breath figures should be in the mean range between 15µm to 150 µm. The inter-well distance can induce distinct effects on biological applications that involve presentation of breath structures. For instance, the inter-well distance will affect the organization and cohesion properties of adhering cell layers with subsequent effects on their capacity to perform inherent biological functions.

Specifically, the water droplet composition can be assisted by the following means in the condensation step 13: decreasing polymer temperature prior to condensation increases the well diameter 71, increases the inter-well distance 73 and the depth 72 of the breath figures; higher vapor pressure and/or temperature increases well diameter 71, inter-well distance 73 and well depth 72.

The more extended the final curing step 14 leading to a larger Young's modulus and higher viscosity of the liquid polymer prior to the condensation of droplets, the larger the diameter, the inter-well distance, but smaller the well depth of the breath figures.

As mentioned above, the condensation conditions in condensation step 13 and the curing conditions in curing step 14 directly influence the distribution of the wells after evaporation of the water droplets 40 and shown in Fig. 2C and 3C.

Treatment step 15 is relating to applying the cured polymer with its specific topography on substrates of various chemistries, 2-D and 3-D geometries for biomedical and flow control purpose. Examples of the substrates are electrical drivelines, fluid drivelines, implants, catheters, stents, artificial arteries etc. Different configurations of the device setup are schematically shown in Figure 5.

Fig. 5 shows schematically different configurations of the device setup after the final treatment step 15. Reference numeral 80 as in portion (A) is relating to the substrate structure which can be the substrate 51 as structure used to prepare the cured polymer layer 23, but it can also be the application structure to which the cured polymer structure 23 is transferred. Said cured polymer structure 23 is shown in the portions (B), (C.1), (C.2), (C.3), (D.1) and (D.2) with reference numeral 81.

So portion (B) of Fig. 5 shows a substrates of various chemistries and 2D and 3D geometries as suitable for the support of a layer of solid elastomer with topographies of tuneable breath figures 81. Examples of the substrates are electrical drivelines, fluid drivelines, implants, catheters and stents, and cardiovascular and dermatological equipment/accessories. The layer of solid elastomer 81 (with wells) can be further physically and/or chemically modified for the functional display of proteins, small molecules, and synthetic polymers via physical and/or chemical attachment as shown with chemically modified breath structure 82 in (C.1), i.e. such molecules are adhering to the inside of the different wells in different concentration. Solid elastomers can be used to influence the adhesion, culture and phenotype of different types of mammalian cells (e.g. endothelial, mesenchymal, epithelial, stem cells) with the reference numeral 83 independently as in (C.2) or together with the presentation of biological proteins, synthetic and biological hydrogels, and small molecules resembling tissue-like conditions with the reference numeral 82 in (C.3).

The different systems emanating from the breath topography (B), (C.1), (C.2) and (C.3) can instruct biological processes, such as endothelialization, wound healing, cell migration, proliferation, under in vitro or in vivo (and ex vivo) conditions or various of biological tissues (human body). Such biological processes initiated through the topography are symbolized with the rectangle with tissue elements 85 shown in (D.1). These can be subject to different profiles of physiological and non-physiological flow as indicated with arrow 86 in (D.2).

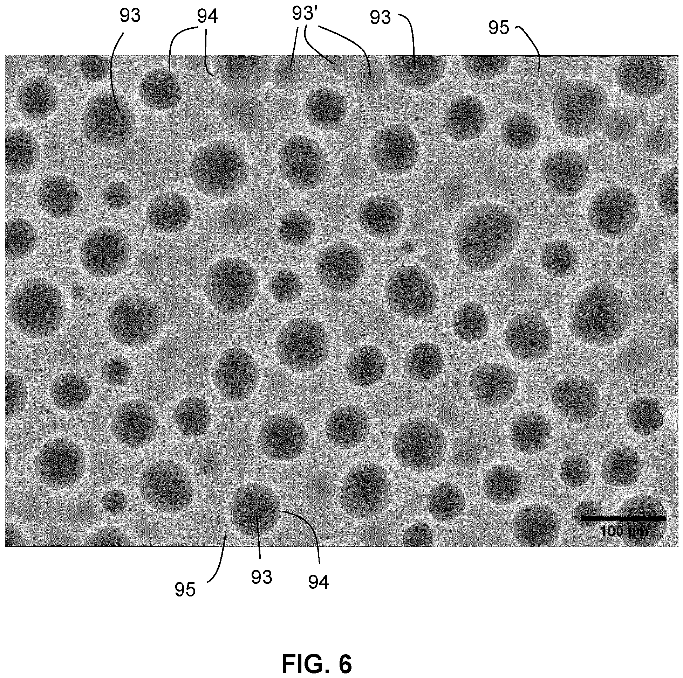

Fig. 6 finally shows a microscopic image of a patterned surface after application of the invention. The darker spots are the polymer surfaces 93 inside a well, having a higher edge showing as a lighter surrounding 94 and inter-well surfaces 95. It can be seen that there are also at the same time shallow well surfaces 93' with a less deep impression and also a less high edge around. The size of the average well crater is about 50 micrometres with some being small as 10 micrometre and other larger up to 80 micrometers. The size of the craters can influence the properties of the material upon which the breath structures are formed following our method. For instance, the size of craters and their inter-well distance can affect the physical adhesion with contacting surfaces. The presence of smaller and larger wells broadens the application potential of the surfaces produced after our method. For instance, it can be used for the separation of objects and particles of different sizes in combination with size-mediated physical, chemical, and biological interactions.

**LIST OF REFERENCE SIGNS**

| | | | |
|---|---|---|---|
| 10 | mixing step | 61 | γ_{w} |
| 11 | application step | 62 | γₛ |
| 12 | initial partial curing step | 63 | γ_{sw} |
| 13 | condensation step | 71 | well diameter |
| 14 | final finishing curing step | 72 | well depth |
| 15 | chemical or biological treatment step | 73 | interwell distance |
| 21 | liquid polymer | 80 | substrate |
| 22 | partially cured polymer | 81 | breath figure structure, layer of solid elastomer (with wells) |
| 23 | cured polymer | 82 | chemically modified breath structure |
| 31 | polymer surface (before water deposition) | 83 | mammalian cells |
| 32 | polymer surface outside well | 85 | biological growth processes as shown |
| 33 | polymer surface inside well | 86 | flow direction |
| 34 | well edge (crater edge) | 93 | polymer surface inside well |
| 35 | inter well surface | 93' | shallow well surface |
| 40 | water droplet | 94 | well edge |
| 41 | environment, space above the polymer surface | 95 | inter well surface |
| 41' | humid environment | | |
| 51 | substrate | | |
| 52 | target surface (substrate surface) | | |

## Claims

1. Fabrication method of an elastomer breath structure comprising the steps:
- applying (11) a liquid elastomer (21) onto a substrate (51),
- initial curing (12) the liquid elastomer (21) on the substrate (51) partially into a partially cured polymer (22),
- condensation (13) of water droplets (40) from the environment (41') onto the partially cured polymer (22), and
- finishing curing (14) of the partially cured polymer (22) with parallel or sequential removal of the water droplets (40) creating surface dimples (33) on the cured elastomer (23).

2. Fabrication method according to claim 1, wherein the condensation step (13) comprises controlling the original environment (41) of the partially cured polymer as a humid chamber atmosphere (41') with a relative humidity of between 20 and 100%, especially 40% and 100% and further especially 60 to 90%.

3. Fabrication method according to any one of claims 1 to 2, wherein the humid chamber atmosphere (41') is controlled that the surface covered by water droplets (40) is between 10% and 80%, especially 20 to 50%, of the polymer covered substrate surface.

4. Fabrication method according to any one of claims 1 to 3, wherein the initial curing step (12) of the liquid elastomer (21) on the substrate (51) into a partially cured polymer (22) is configured to obtain a partially cured polymer (22) with a surface tension such that condensed water droplets (40) sink in into the previous undisturbed surface (21) by a depth of 1 to 100% before the finishing curing step (14) removes the sunk in water droplets (40).

5. Fabrication method according to claim 4, wherein the aspect ratio of well width (71) to well depth (72) is between 30 to 1 and 10 to 1.

6. Fabrication method according to any one of claims 1 to 5, wherein the parallel or sequential removal within the finishing curing step (12) is performed as an evaporation step, a washing step or a centrifugation step.

7. Fabrication method according to any one of claims 1 to 6, wherein the humidity and temperature of the humid environment (41') is controlled within the condensation step (13) and the finishing curing step (14).

8. Fabrication method according to any one of claims 1 to 7, wherein the application step is preceded by a step of:
- mixing (10) one or more liquid polymers.

9. Fabrication method according to claim 8, wherein the mixing step (10) comprises mixing of one or more liquid polymers, especially room temperature vulcanizing silicones or silicones curing at temperatures between -10 and +60 degrees Celsius, optionally with polymers and copolymers having a different degree of miscibility.

10. Fabrication method according to any one of claims 1 to 9, wherein the application step (11) of the liquid polymer (21) on the substrate (51) is conducted by a process from the group encompassing spin-coating, dip-coating, air spray or 3D-printing.

11. Fabrication method according to any one of claims 1 to 10, wherein the finishing curing step (14) is followed by a chemical or biological treatment step (15) to apply predetermined chemical compounds or biological cells into the surface dimples (33) and/or on the in-between dimple surface (35).

12. Patterned surface of a polymer, fabricated according to any one of claims 1 to 11, having an aspect ratio of well width (71) to well depth (72) between 30 to 1 and 10 to 1.

13. Percutaneous driveline coated at least adjacent to the percutaneous portion with a patterned surface according to claim 12, wherein predetermined chemical compounds or biological cells are in the surface dimples (33) and/or on the in-between dimple surface (35).
